Europäisches Patentamt

European Patent Office

Office européen des brevets

(11) Publication number: **0 131 096**
**A2**

(12) **EUROPEAN PATENT APPLICATION**

(21) Application number: **84104401.9**

(22) Date of filing: **18.04.84**

(51) Int. Cl.⁴: **G 01 N 33/53**

(30) Priority: **02.05.83 US 490306**

(43) Date of publication of application:
**16.01.85 Bulletin 85/3**

(84) Designated Contracting States:
**CH DE FR GB IT LI**

(71) Applicant: **MILES LABORATORIES, INC.**
**1127 Myrtle Street**
**Elkhart Indiana 46514(US)**

(72) Inventor: **Dean, Kenneth James**
**26921 Dumbarton Court**
**Elkhart, IN 46514(US)**

(72) Inventor: **Thompson, Stephan George**
**522 Manchester Drive**
**South Bend, IN 46615(US)**

(74) Representative: **Adrian, Albert, Dr. et al,**
**c/o BAYER AG Konzernverwaltung RP Patentabteilung**
**D-5090 Leverkusen 1, Bayerwerk(DE)**

(54) **Homogeneous immunoassay method for determining binding substances and reagent system therefor.**

(57) A homogeneous immunoassay method for determining an analyte such as a hormone binding protein or receptor or a specific antibody, wherein the test sample is combined with (i) a macromolecular reagent capable of binding with the analyte and (ii) a labeled antireagent capable of competing with the analyte for binding with the macromolecular reagent. The binding of the labeled antireagent to the macromolecular reagent results in a change in the detectable response generated by the label. The labeled antireagent preferably is a labeled monoclonal antibody or fragment thereof. The method is particularly suited to the determination of iodothyronine uptake in biological fluid test samples.

EP 0 131 096 A2

Homogeneous immunoassay method for determining
binding substances using a macromolecular reagent
and labelled antireagent and a reagent system for
performing the homogeneous immunoassay to determine
an analyte in a test sample

BACKGROUND OF THE INVENTION

1.  FIELD OF THE INVENTION

The development of specific binding assay techniques has provided extremely useful analytical methods
for determining various organic substances of diagnostic, medical, environmental and industrial importance
which appear in liquid mediums at very low concentrations.  Specific binding assays are based on the interaction between the substance under determination, sometimes referred to as analyte, and a binding partner
thereof.  Where the analyte is a hapten or antigen and
its binding partner is an antibody, the assay is known
as an immunoassay.  Other binding interactions between
the analyte and a binding partner serve as the basis
of binding assays, including the binding interactions
between hormones, vitamins, metabolites, and pharmacological agents, and their respective receptors and binding substances.

In situations where the analyte is a binding substance, e.g., a binding protein or receptor such as an
iodothyronine binding protein (thyroxine binding
globulin, thyroxine binding prealbumin, and the like)
or an antigen-specific antibody, it is common to

MS-1285

employ binding assay techniques where a labeled form of the substance that is bound by such binding substance is used. The uptake of the labeled reagent is determined by measuring the amount of label which becomes bound to the binding substance (bound-species) or the remaining free-species of the labeled reagent. Where the labeled reagent in the bound-species is essentially indistinguishable in the presence of the labeled reagent in the free-species by the means used to monitor the label, the bound-species and the free-species must be physically separated in order to complete the assay. This type of assay is referred to in the art as "heterogeneous". Where the bound-species and free-species forms of the labeled conjugate can be distinguished in the presence of each other, a "homogeneous" format can be followed and the separation step avoided.

A variety of labels have been used in assays of this type for determining binding substances. The first labels used were radioisotopes, however, because of the inconvenience and difficulty of handling radioactive materials, assay systems have been devised using materials other than radioisotopes as the label component, including enzyme cofactors, enzyme substrates, enzyme modulators, e.g., activators and inhibitors, cycling reactants, spin radicals, enzymes, bacteriophages, metals and organometallic complexes, organic and inorganic catalysts, enzyme prosthetic groups, chemiluminescent reactants, and fluorescent molecules.

- 3 -

0131096

## 2. DESCRIPTION OF THE PRIOR ART

Representative of the prior art binding assays for determining binding substances based on the uptake of a labeled reagent are the following:

A. Radioassays employing a radiolabeled form of the substance which is the object of binding by the binding substance and requiring a distinct separation step - see U.S. Pat. Nos. 3,376,114; 3,451,777; 3,615,222; 3,710,117; 3,711,247; 3,823,001; 4,211,763; 4,238,471; and 4,252,782. The main disadvantages of these radioassays are the short shelf-life, handling hazards, and instrumentation requirements associated with the use of radioisotope labels.

B. Nonradioisotopic, homogeneous assays employing various nonradioactive labels such as enzyme substrates, enzyme prosthetic groups, enzyme inhibitors, and enzymes - see U.S. Pat. Nos. 4,134,792; 4,190,496; 4,238,565; 4,279,992; and 4,341,865.

It is also known to perform assays for binding substances by setting up a competition between (a) the binding substance under determination and (b) an antibody to a low molecular weight substance bound by such binding substance, for binding to a labeled form of such low molecular weight substance. U.S. Pat. No. 4,168,207 concerns an assay for TBG wherein the sample is combined with antibody to triiodothyronine (T-3) and both labeled and unlabeled T-3. The label employed is an enzyme and the assay is homogeneous since binding of enzyme-labeled T-3 to the antibody results in a measurable inhibition of enzyme activity.

MS-1285

A particularly unique and advantageous homogeneous binding assay employing labeled antibodies is described in U.S. Patent Application Serial No. 359,610, filed March 18, 1982, and assigned to the instant assignee. In one aspect, this assay system employs a single labeled monoclonal antibody reagent. A test sample suspected to contain the analyte of interest is combined with a labeled monoclonal antibody preparation in which antibody to the analyte is substantially the only labeled component. The label employed is selected such that a detectable response is measurably different in a qualitative or quantitative sense when the labeled antibody is bound to the analyte compared to when not so bound. The resulting detectable response is therefore a function of the analyte in the test sample. As the analyte concentration increases, there is increased binding of labeled antibody and thus increased modulation of the detectable response of the label. In one embodiment, the measurable change in the detectable response upon binding of analyte by the labeled antibody is due to steric hindrance of access of a member of a reagent detection system.

A general review of the application of monoclonal antibodies in clinical immunology is provided in *Clin. Chem.* *27*:1797(1981). The review lists several advantages of using labeled antibodies in immunoassays and speculates on a few potential future applications of labeled monoclonal antibodies to assay systems based on proximal binding of labeled antibody pairs. The suggestion is to substitute labeled monoclonal antibodies for conventional polyclonal antibody preparations in those known assay systems employing two different labeled antibodies.

## SUMMARY OF THE INVENTION

The present invention provides a homogeneous immunoassay involving a unique set of reagents including a labeled antibody, or antibody fragment, for determining analytes such as binding substances in a test sample. Such reagents comprise (i) a macromolecular reagent capable of binding with the analyte, and (ii) a labeled antireagent, e.g., an antibody, capable of binding to the macromolecular reagent in a manner which is competitive with the binding of analyte with such macromolecular reagent. The label comprised in the labeled antireagent provides a detectable response which is measurably different when the labeled antireagent is bound with the macromolecular reagent compared to when not so bound. In a preferred embodiment, the reagent system also includes a chemical detection system for the label. In such embodiment, the label and detection system are selected such that the label and a member of the detection system interact to provide a detectable response which is different when the labeled antireagent is bound compared to when not bound due to steric hindrance of access of the detection system member to the label.

The detectable response is then measured as a function of the analyte in the sample. The more that the analyte binds the macromolecular reagent, the less labeled antireagent is able to bind such reagent, resulting in the generation of the response according to the amount of unbound labeled antireagent. Where, as is the usual case, binding of the labeled antireagent with the macromolecular reagent results in a decrease in the amount of response generated, as more analyte binds the macromolecular reagent, the more response is generated.

MS-1285

In a preferred embodiment, the analyte is a class of serum binders, principally TBG, for the iodothyronine hormones, e.g., thyroxine (T-4) and triiodothyronine (T-3). Thus, for determining iodothyronine uptake of a biological sample, the macromolecular reagent comprises a conjugate of an iodothyronine with a macromolecular carrier material and the labeled antireagent comprises a labeled antiiodothyronine. The present method is also applicable to the determination of a variety of other binding substances, including other hormone binding proteins, tissue hormone receptors, and specific immunoglobulins.

The labeled antireagent is most preferably a labeled monoclonal antibody or fragment thereof, i.e., a labeled form of an antibody, or its fragment, obtained by somatic cell hybridization techniques which yield essentially chemically pure immunoglobulin preparations. In preparing monoclonal antibody preparations for labeling, nonspecific proteins are readily and efficiently removed by subjecting the monoclonal preparation to a separation technique selective for immunoglobulins. Since substantially all immunoglobulins in the preparation will be monoclonal, i.e., chemically identical, the separated immunoglobulin fraction will be substantially free of nonspecific components. Therefore, the resulting labeled monoclonal antireagent will exhibit a change in detectable response essentially only upon binding the macromolecular reagent. The use of labeled monoclonal antibodies in this way significantly reduces the background response that would otherwise be obtained when using labeled polyclonal antibodies, i.e., antibodies of mixed structure and origin resulting from conventional antiserum techniques.

MS-1285

## DESCRIPTION OF THE PREFERRED EMBODIMENTS

*ANALYTE*

In its broadest sense, the present invention applies to the determination of any substance which has a specific binding affinity for a second substance, provided that an antibody can be prepared against such second substance and can compete with the analyte for binding to such second substance. The analyte can be any appropriate organic molecule, e.g., peptide, protein, carbohydrate, glycoprotein, steroid, and the like, and in functional terms usually will be an antigen, hapten, antibody, hormone, vitamin, metabolite, or drug, or a binding partner or receptor therefor. The present method is particularly applicable to the determination of binding substances such as binding proteins and receptors, including thyroxine binding globulin (TBG), thyroxine binding prealbumin, cortisol binding globulin, estrogen receptors, ACTH receptors, avidin, cyanocobalamins, enzymes, insulin receptors, endorphin receptors, norepinephrin receptors, serotonin receptors, prostaglandin receptors, lectins, and antibodies, e.g., antibodies specific for particular antigens or haptens such as viruses (hepatitis virus, CMV, rubella), allergens, Rh factors, and drugs (penicillin and the like), and autoimmune antibodies.

MS-1285

- 8 -    0131096

In principle, the analyte is not limited to any particular molecular weight range, however, when applied to the determination of binding proteins and receptors, as a practical matter, the molecular weight range of the analyte will be between about 10,000 and 10,000,000 daltons, more usually between 25,000 and 250,000 daltons. Similarly, the binding partner which specifically binds with the analyte is not limited as to its molecular weight.

In one aspect, the analyte which is the object of the present assay method comprises a class of related substances which have a specific binding affinity for a particular substance or molecule or a class of related substances or molecules (ligand). Thus, in essence, one can apply the present method to determining the capacity of a test sample to bind or take up such ligand. Stated another way, one can determine the extent of unsaturation of ligand-binding sites on components of the test sample. For example, one can determine the extent of unsaturation of hormone binding proteins in a biological test sample, such as serum or plasma, where the ligand is a hormone such as an iodothyronine (T-3 or T-4). The value determined in such an assay is sometimes expressed as an uptake index, e.g., T-3 uptake or T-4 uptake.

MS-1285

## MACROMOLECULAR REAGENT

This component of the reagent system is characterized by its ability to be bound by the analyte and the antireagent. The macromolecular reagent will bear one or more sites which will accommodate the binding of either said analyte or the antireagent, but not both simultaneously. In this way, a competition can be established between the analyte and labeled antireagent for binding to such binding sites on the macromolecular reagent, with the amount of label which becomes affected by binding of labeled antireagent to the macromolecular reagent being a function of the amount of competing analyte present.

The macromolecular reagent will be characterized by its ability to effect modulation of the detectable response produced by the label in the labeled antireagent upon binding therewith. Such modulation will normally be caused by the size of the reagent and/or the presence of label-modulating elements on the reagent as will be described more fully below. Within the above parameters, the macromolecular reagent can take a wide variety of forms. This reagent will have a minimum size of about 5,000 daltons in order to be capable of inducing steric hindrance of the label upon binding with the labeled antireagent and/or of carrying label-modulating elements. Usually, the macromolecular reagent will have a molecular weight greater than about 10,000 daltons, and preferably greater than 50,000 daltons. The minimum size of the reagent useful in a given reagent system will be dependent upon the exact nature of the labeled antireagent selected and the desired performance characteristics of the assay, e.g, sensitivity. The maximum size of the macromolecular reagent is essentially unlimited and will be selected based strictly on the preferences of the designer of the reagent system.

MS-1285

Where there exists in nature a substance which will bind with the analyte, for which an antireagent can be prepared, and which is sufficiently macromolecular (greater than 5,000 daltons) such substance itself can serve as the macromolecular reagent. Such a situation often is encountered in biological systems wherein the analyte is a binding protein or receptor for a relatively large molecule such as certain protein hormones, e.g., chorionic gonadotropin, insulin, growth hormone, follicle-stimulating hormone (FSH), luteinizing hormone (LH), thyroid-stimulating hormone (TSH), and prolactin. Likewise, an antigen can be used as the macromolecular reagent where the analyte is an antibody, for example, in the case of determining antibodies specific to certain viruses, allergens, Rh factors, and the like. Furthermore, the macromolecular reagent can be an antibody, or fragment thereof, which binds to the analyte, which in such case will be a relatively low molecular weight hapten (e.g., 100-1500 daltons) or an antigen (e.g., 1000-10,000,000 daltons). In such a situation, the antireagent will be selected from antibodies raised against the antianalyte antibody, or fragment, and which will bind at or sufficiently near the combining site of the antianalyte so as to significantly inhibit binding of both analyte and antireagent to the antianalyte macromolecular reagent simultaneously. The antireagent can be an antiidiotipic antibody or the like and can be conveniently prepared by monoclonal techniques.

Alternatively, or in a case where the analyte has a specific binding affinity for a relatively small molecule, e.g., less than about 2000 daltons, one can prepare a suitable macromolecular reagent by forming a polymer or aggregate of such ligand or a conjugate of such ligand with a macromolecular carrier. Such a conjugate will comprise one or more ligands bound to a carrier molecule of molecular weight at least

MS-1285

greater than 5,000 daltons, usually greater than 10,000 daltons, and preferably greater than 50,000 daltons. The ligands will be bound to the carrier in such a manner that the conjugate is substantially stable under the conditions of the assay. In the usual case, the ligands will be chemically linked by covalent bonds to the carrier molecule. The wide variety of materials that can serve as the carrier material and the wide variety of methods for bonding the appropriate ligand or ligands thereto are well within the skill of the ordinary worker in the field.

The carrier material can be soluble or insoluble in the test sample and reaction mixture, although it will generally be preferable to employ soluble, usually water soluble, carrier materials which can be uniformly dispersed in the reaction mixture. However, even insoluble, porous or nonporous, materials in the form of resins, gels, plastic strips, bibulous pads, and even the surface of the reaction vessel itself, can serve as the carrier for the macromolecular reagent. The shape of the carrier material is not critical and is primarily a matter of choice for the designer of the reagent system.

Clearly, an extremely wide variety of materials can serve as a macromolecular carrier since its principle purpose is simply to impart a minimum size to the molecule which is the object of the competitive binding between the binding substance analyte and anti-reagent. A brief, but certainly not exhaustive, list of materials which can be used as the carrier material includes proteins (e.g., albumins and globulins), polypeptides, polysaccharides, synthetic and naturally occurring organopolymers such as celluloses, including modified celluloses such as carboxymethylcellulose, hydroxypropylcellulose, and DEAE-cellulose, starches, agar, agarose, dextran, gelatin, polyvinylalcohols, polyethyleneimine,

MS-1285

synthetic poly(amino acids) such as polylysine, polyglutamic acid, and the like. At least one, and normally several, ligands are bound to the carrier material. The number of ligands bound to a particular carrier material will vary from 1 to the number of linking sites available on the carrier according to the needs of the assay involved.

## *LABELED ANTIREAGENT*

Binding of the macromolecular reagent to labeled antireagent results in a measurable change, either a decrease or an increase, in the detectable response produced by the label. Preferably, the label is selected to provide a sterically sensitive detectable assay response upon interaction, e.g., chemical reaction, with a member of a chemical detection system comprising one or more substances which interact with the label to generate the measured signal. In such assay systems, the masses of the labeled antireagent and the macromolecular reagent will be selected such that the mass of the labeled antireagent will be significantly increased upon binding of the macromolecular reagent. As the relative mass of the macromolecular reagent becomes larger, the steric effects in the environment of the label become more pronounced, and thus, more readily detectable.

In the usual case, the label itself will preferably be small, e.g., of molecular weight less than 50,000, generally less than 10,000, more usually less than 4,000, and preferably less than 2,000, and the detection system member or members with which it interacts will preferably be significantly larger, e.g., more than 3 times larger, more usually 10 times larger, and preferably 20 to 100 times or more larger than the label. Accordingly, in the most preferable systems with labels having masses of the order of 100 to 2,000 daltons, at least one member of the detection system

MS-1285

with which the label must interact to provide the detectable signal will be of the order of 10,000 to 200,000 daltons or greater. Such size relationship between the label and the detection system member increases the probability of a significant steric effect upon binding of macromolecular reagent with the labeled antireagent. Preferred labels therefore are participants in an enzyme-catalyzed reaction, such as enzyme substrates, coenzymes, enzyme prosthetic groups, and enzyme inhibitors, since a wide variety of enzymic reactions are available from which to chose assay components. Many small substrates, coenzymes, and inhibitors are known for enzymes of sufficiently large molecular weight to have the preferred size relationship between label and its interacting detection system member. This applies likewise for prosthetic groups and their corresponding apoenzymes. Similarly, many enzymes are known which have significantly larger substrates or for which artificially large substrates, coenzymes, or inhibitors can be prepared by coupling small substrates, coenzymes, and inhibitors to high molecular weight backbone materials such as water soluble polymers.

As alternatives to, or in combination with, the steric effects on the label, one can design the macromolecular reagent with which the labeled antireagent binds to comprise label-modulating elements, i.e., chemical groupings or components which interact with the label when proximate thereto to modulate the detectable response. Such proximal effects are well known in the art of homogeneous binding assays, for example, see *Clin. Chem. 27*:1797-1806(1981). For instance, one of the antireagent and the macromolecular reagent can comprise a light emitting element, e.g., a fluorescent, chemiluminescent, or phosphorescent group, and the other can comprise an appropriate light absorbing element that quenches and/or reemits light of a

MS-1285

distinct character. Commonly, such systems will involve fluorescence or chemiluminescence quenching [see
U.S. Pat. No. 4,160,016 and *J. Clin. Path. 30*:526(1977)],
or energy transfer [see U.S. Pat. No. 3,996,345].
Fluorescence polarization can also be utilized where
one of the antireagent and macromolecular reagent
comprises a fluorescer and the other is of a sufficiently larger size that a fluorescence polarization difference can be detected upon binding of the two assay components. [see *J. Exp. Med. 122*:1209(1975) and *Immuno-
chem. 7*:799(1970)]. Another proximal effect approach
involves enzyme channeling and the like where one of
the antireagent and macromolecular reagent comprises
one component of an enzyme system and the other comprises a second component of such enzyme system wherein
some product of the enzymatic reaction is produced at a
rate dependent upon the proximal relation of the two
enzyme system labels. Such enzyme system labeling pairs
can involve, for example, two different enzymes where
the product of a reaction catalyzed by one is a substrate for the other, an enzyme and its substrate or
a cofactor or inhibitor, or an apoenzyme and its
prosthetic group [see U.S. Pat. No. 4,233,402].

Where the modulation effect is due to steric
hindrance, the label comprised in the antireagent
can be any substance which interacts with another
molecule or molecules (a member of the chemical
detection system) to give a detectable response,
where such interaction is sterically sensitive, that
is where binding of the macromolecular reagent to the
labeled antireagent sterically hinders the response-
producing interaction. Specifically preferred labels
are those described in detail in the aforementioned
U.S. Patent Application Serial No. 359,610, and briefly
include enzyme substrate and coenzyme labels, U.S. Pat.
No. 4,279,992 and U.K. Pat. Spec. 1,552,607; enzyme

MS-1285

prosthetic group labels, U.S. Pat. No. 4,238,565; enzyme modulator (e.g., inhibitor) labels, U.S. Pat. Nos. 4,134,792 and 4,273,866; enzyme labels, U.S. Pat. Nos. 3,817,837 and 4,043,872; chemically-excited fluorescent labels, U.S. Pat. No. 4,238,195; and epitope labels, U.S. Pat. Nos. 3,935,074 and 3,998,943.

The steric effects produced by the binding of the macro-molecular reagent and the labeled antireagent are enhanced by selecting antireagents having masses that will be significantly increased by complexation with the macromolecular reagent. In general, the antireagent preferably will be smaller than 10 times the mass of the reagent, more usually smaller than the absolute mass of the reagent, and more preferably smaller than 0.25 times the mass of the reagent. The mass of the antireagent can be decreased by selecting antibodies of an immunoglobulin class which have relatively low molecular weights, e.g., IgG antibodies have molecular weights around 150,000 whereas those of the IgM class have molecular weights around 900,000. Also, one can selectively cleave antibodies to give fragments of lower molecular weight which retain their specific binding affinity, e.g.. various fragments of IgG antibodies can be prepared such as Fab (50,000 daltons), F(ab') (53,000 daltons), and $F(ab')_2$ (106,000 daltons).

In all embodiments, antireagent which is labeled is an immunologically-derived binding substance capable of binding with the macromolecular reagent in competition with the analyte. The antireagent normally comprises whole antibodies, or appropriate fragments or aggregates thereof, raised against the binding sites (epitopes) on the macromolecular reagent to which the analyte also binds. Thus, where the macromolecular reagent comprises an antigen or other naturally occurring macromolecular ligand to which the analyte

MS-1285

binds, antireagent is raised against such ligand. The resulting antibodies are screened for competition with the analyte for binding to such ligand. Where the macromolecular reagent comprises a conjugate of ligands with a macromolecular carrier as described above, antireagent is raised against the ligand, or if haptenic, against a ligand-immunogenic carrier conjugate as is known in the art.

When in the form of whole antibody, antireagent can belong to any of the classes and subclasses of known immunoglobulins, e.g., IgG, IgM, IgE, and so forth. Any fragment of any such antibody which retains specific binding affinity for the macromolecular reagent can also be employed, for instance, the fragments of IgG conventionally known as Fab, F(ab'), and F(ab')$_2$. In addition, aggregates, polymers, and conjugates of immunoglobulins or their fragments can be used where appropriate. Such poly(antireagent) can be prepared in any available manner so as to maintain binding affinity for the reagent. Other forms of antireagent can be employed so long as the material selected has a specific binding affinity for the reagent concerned.

The immunoglobulin source for the antireagent can be obtained in any available manner. Usually, antireagent immunoglobulin will be obtained by conventional antiserum techniques or monoclonal techniques. Antiserum containing antireagent is obtained by well-established techniques involving immunization of an animal, such as a rabbit, guinea pig, or goat, with an appropriate immunogen. State-of-the-art reviews are provided by Parker, *Radioimmunoassay of Biologically Active Compounds*, Prentice-Hall (Englewood Cliffs, New Jersey, U.S.A., 1976), Butler, *J. Immunol. Meth. 7:* 1-24 (1975); Weinryb and Shroff, *Drug Metab. Rev. 10:* 271-283 (1975); Broughton and Strong, *Clin. Chem. 22:* 726-732 (1976); and Playfair et al, *Br. Med. Bull. 30:* 24-31 (1974).

MS-1285

Monoclonal antireagent is preferably used for the reasons stated previously. Monoclonal antibodies are chemically homogeneous and are obtained by somatic cell hybridization techniques; see *Lymphocyte Hybridomas*, ed. Melchers et al, Springer-Verlag (New York 1978) and *Methods in Enzymology 73 (Part B)*:3-47(1981). In general, monoclonal antireagent immunoglobulin is produced by fusing lymphocytes which produce such antibody with nonsecreting myeloma cells to form hybridomas, isolating a hybridoma clone which secretes the desired antibody, and harvesting the secreted monoclonal antibody. The lymphocytes involved in hybridization are commonly spleen cells removed from an animal such as a mouse or a rat which has been immunized in the conventional manner against the pertinent epitopes on the macromolecular reagent.

The selected label is linked in any conventional manner to the antireagent to form the labeled component of the present invention. A wide variety of means for linking the two elements are available and known in the art. The linkage can be covalent and involve a single chemical bond or a chain comprising from 1 to 50 atoms, more commonly 1 to 30 atoms, and usually 1 to 20 atoms, excluding hydrogen, principally composed of carbon and heteroatoms selected from nitrogen, oxygen, phosphorous, and sulfur. Conventional linking groups are described at length in the literature. See for example U.S. Pat. Nos. 4,230,797; 4,279,992; 3,817,837; 3,935,074; and 3,996,345. The linking group can be comprised of a side arm group put on the label for the purposes of spacing and/or functionalizing the label for coupling to the antireagent and/or the residue from a bifunctional coupling agent used in linking the label or derivatized label to the antireagent.

MS-1285

Since the label is associated with antireagent in the present invention, it is necessary to achieve a relatively high degree of purity for the antireagent preparation which is subjected to labeling conditions or else significant nonspecific labeling can occur resulting in background label responses. Affinity purified conventional antiserum can be used, but monoclonal antibody preparations are especially preferred.

Once a monoclonal antireagent preparation (ascites fluid or tissue culture fluid) has been obtained, a fraction containing essentially only immunoglobulin is separated from the other proteins that may be present. Such separation can be accomplished in any available manner. Preferably, affinity chromatography techniques are applied to this task. As affinity binding partner on the chromatography column can be used anti-(antireagent) which usually will be an antibody to the class of immunoglobulin to which the desired antireagent belongs, e.g., anti-IgG, or a fragment thereof. While not a preferred method, one can also use the reagent itself or the ligand as the affinity binding partner. A preferred affinity chromatography technique employs the substance commonly referred to as protein A, a protein obtained from *Staphylococcus aureaus* which has the unique property of binding specifically to IgG immunoglobulins [*J. Immunol.* *97*:822(1966) and *Immunol.* *103*:828(1969)]. Protein A is commercially available as the isolated protein or attached to gel particles suitable for use as an affinity chromatography column matrix [Protein A-Sepharose, Pharmacia Fine Chemicals, Piscataway, New Jersey, USA]. The use of protein A to separate antireagent from the monoclonal preparation is particularly advantageous. Since the only immunoglobulin present in the monoclonal preparation is antireagent, one need only apply techniques that separate immunoglobulins and it is assured that only antireagent will be isolated.

The purified antireagent preparation is then labeled according to the particular detection system that is desired. The label, as described above, is coupled to antireagent in any available manner. Since the only protein substantially present in the purified antireagent preparation is antireagent, the labeling substance can be efficiently and specifically attached to antireagent therein by reaction under conditions that form covalent bonds between the labeling substance and proteins in general. This ability to use standard protein modification reactions to label antireagent without concern for nonspecific labeling of proteins is particularly advantageous.

One or more labels can be attached to an individual antireagent molecule and, where the nature of the label allows, *vice versa*. Ordinarily, the antireagent will carry between 1 and 30, more usually between 1 and 20, labels per molecule of antireagent. While it is desirable to have multiple labels per antireagent to enhance sensitivity, too high a labeling density can affect the ability of antireagent to effectively bind with reagent. In the case where the label is relatively large and polyfunctional, such as an enzyme, multiple antireagents can be attached to a single label. Binding to the reagent leads to the formation of lattices due to bonding of a single labeled antireagent conjugate to multiple reagent molecules, each of which in turn can be bound by labeled antireagent, leading to increased steric hindrance at or near the label.

### *REACTION MIXTURE AND CONDITIONS*

The test sample to be assayed can be a naturally occurring or artificially formed liquid suspected to contain the analyte, and usually is a biological fluid or a dilution thereof. Biological fluids that can be assayed include serum, plasma, urine, saliva, milk, and amniotic and cerebrospinal fluids.

The assay reaction will in almost all cases be allowed to proceed under mild conditions. The order of addition of the macromolecular reagent and labeled antireagent, and where used, the detection system, is not critical, provided that the complete detection system and label are not permitted to generate significant response in the absence of sample and the reagent. In general, the reaction mixture will be an aqueous medium with any desirable organic cosolvents being present in minor amounts. The temperature of the reaction will be maintained at a constant level in normal circumstances throughout the incubation period and the measurement step. Temperatures will generally be between 5 and 50°C, more usually between 20 and 40°C. Preferably, the reaction will proceed at room temperature. The pH of the reaction mixture will vary between 5 and 10, more usually between 6 and 9. The concentration of various reagents will depend on the level of analyte expected in the test medium, with such level usually being between $10^{-3}$ and $10^{-12}$M. As in the case of the previously described reaction parameters, selection is primarily based on empirically derived optimization balanced against the preferences and needs of the technician who will ultimately perform assays on a routine basis. None of the parameters therefore is of a critical nature to the present invention, rather they are all within the ordinary skill in the art.

MS-1285

## REAGENT SYSTEM

The reagent system, i.e., reagent combination or means, of the present invention comprises all of the essential chemical elements required to conduct a desired assay method encompassed by the present invention. The reagent system is presented in a commercially packaged form, as a composition or admixture where the compatibility of the reagents will allow, in a test device configuration, or as a test kit, i.e., a packaged combination of one or more containers holding the necessary reagents. Included in the reagent system are the macromolecular reagent and labeled antireagent, and, where appropriate, the detection system for the label. Such binding reaction reagents can include, in addition, any other optional reagents for performing the particular assay technique involved. Of course, the reagent system can include other reagents as are known in the art and which may be desirable from a commercial and user standpoint, such as buffers, diluents, standards, and so forth. Also preferred is a test device comprising the reagent system and a solid carrier member incorporated therewith. The various forms of such test device are described in application Serial No. 202,378, filed October 30, 1980, and published as European Pat. Appln. No. 51,213, which is incorporated herein by reference.

MS-1285

- 22 -　0131096

The present invention will now be illustrated, but is not intended to be limited, by the following examples.

EXAMPLES

A. Purification of Monoclonal Antibody to Gentamicin

Mouse ascites fluid containing monoclonal antibody to gentamicin (Scripps-Miles, Inc., LaJolla, California, USA) was purified by affinity chromatography on Protein A-Sepharose as follows.

The ascites fluid [1 milliliter (ml)], stored at -20°C prior to use, was applied to a 4 ml (1 cm x 5 cm) column of Protein A-Sepharose-4B (Pharmacia Fine Chemicals, Piscataway, New Jersey, USA) equilibrated with 20 mM Bicine [N,N-*bis*-(2-hydroxyethyl)glycine], pH 8.2, containing 0.04% sodium azide at room temperature. The column was washed with 15 ml of this Bicine buffer, followed by 15 ml of the same Bicine buffer containing 0.1 molar (M) sodium chloride (NaCl) and then 15 ml of Bicine buffer with no NaCl. The antibody was eluted by washing the column with 0.1 M glycine-hydrochloric acid (HCl) buffer, pH 3.0. Test tubes receiving this acid eluate contained 100 microliters (μl) of 1 M Bicine, pH 8.5, to raise the pH immediately. The column was finally washed with 15 ml of Bicine buffer, to return it to pH 8.2. The absorbance at 280 nanometers ($A_{280}$) of the 100-drop fractions was monitored and fractions containing the antibody were pooled and concentrated to about 1 ml using a Millipore CX-10 immersible ultrafiltration device.

MS-1285

The concentrated antibody solution was chromatographed on Sephadex G25 (fine) (Pharmacia) to remove glycine. The antibody solution (about 1 ml) was applied to an 80 ml (1.5 cm x 45 cm) Sephadex G-25 (fine) column equilibrated with 20 mM Bicine, pH 8.2, containing 0.04% sodium azide at room temperature. The $A_{280}$ of the 100-drop fractions was monitored and fractions containing the antibody were pooled and concentrated, as before, to 2 ml.

    B.  Labeling of Purified Monoclonal Antibody

    N-6-(6-aminohexyl)-7-β-galactosylcoumarin-3-carboxamide (AH-GU) (U.S. Pat. No. 4,259,233) (5 mg, 10 µMol) was dissolved in 200 µl of $H_2O$. Dimethyl-adipimidate (5 mg, 20 µmol) was dissolved in 400 µl of 1 M triethylammonium bicarbonate (TEAB), pH 9.6, and 100 µl of the AH-GU solution at room temperature for about five minutes. The antibody solution [2 ml, 2.4 mg protein as determined by the Lowry method, *J. Biol. Chem. 193*:265(1951)] was then added, and the reaction mixture incubated at room temperature for about one hour. At this time, 100 µl of 1 M glycine-sodium hydroxide (NaOH), pH 9.6, was added to terminate the reaction. The yellow solution was applied to the Protein A-Sepharose column and the GU-labeled antibody to gentamicin was isolated as described above. The GU-antibody was characterized by measuring $A_{343}$ to determine the concentration of GU substituents present ($\varepsilon_{343}^{1mM}$ = 21); $A_{400}$ following hydrolysis with β-galactosidase, to determine how many GU substituents could be hydrolyzed ($\varepsilon_{400}^{1\ mM}$ = 35); the fluorescence was measured to determine how many of the hydrolyzed GU substituents were quenched.

C.  Preparation of Gentamicin-Macromolecular
    Reagent

Bovine serum albumin (BSA, 300 mg) was dissolved in 1.5 ml water. An aliquot (1.0 ml) was added to 2.0 ml aqueous solution of gentamicin sulfate (Schering Corp., Bloomfield, New Jersey, USA) having a pH which had been adjusted to about 4.5 by addition of 2 N hydrochloric acid (HCl) or 2 N sodium hydroxide (NaOH) as needed. The pH of the mixture was adjusted similarly to 4.5. Water was added to give a final volume of about 8 ml and the pH again adjusted to 4.5. After cooling in an ice bath for 15 minutes, 600 mg of 1-ethyl-3-(3-dimethylaminopropyl)carbodiimide hydrochloride (Pierce Chemical Co., Rockford, Illinois, USA) was added to the solution and mixed in the ice bath for 2 hours. After overnight incubation at 4°C, the solution was allowed to come to room temperature and then applied to a column (2.5 x 55 cm) of G-25 (fine) Sephadex (Pharmacia) equilibrated with 50 mM Bicine-0.1% azide, pH 8.5. Seven (7) milliliter fractions were collected and tested for the presence of BSA-gentamicin reagent by positive ninhydrin reaction and absorbance at 280 nm. Fractions containing BSA-gentamicin were pooled.

D.  Assay Protocol - Determination of Goat
    Anti-gentamicin

Increasing amounts (0-40 μl) of goat antiserum to gentamicin (analyte) were added to 1.35 ml of 20 mM Bicine, 0.91% sodium azide, 12 mM magnesium chloride, and 4% polyethylene glycol (6000 MW-Sigma Chemical Co., St. Louis, MO, USA), pH 8.2 containing 200 micrograms (μg) of the BSA-gentamicin macromolecular reagent, *supra*. After a 15 minute incubation at room temperature, 100 μl of labeled monoclonal anti-gentamicin (0.01 $A_{343}$ units) was added, mixed, and incubated for an

MS-1285

additional 15 min. Lastly, 10 µl of 0.5 units/ml of β-galactosidase was added to initiate fluorescence production. After 30 min., the fluorescence was read.

E.   Results

The labeled monoclonal antibody preparation was found to have 0.72 AH-GU substituents per antibody molecule. When the label was incubated with excess β-galactosidase, 0.18 AH-GU substituents (25%) per antibody molecule were hydrolyzed and 0.003 substituents (25%) per antibody molecule (2% of hydrolyzed groups) were detected by fluorescence. The relief of inhibition of GU-antigentamicin hydrolysis by goat antiserum to gentamicin is provided in the table below:

| µL Goat Anti-Gentamicin | Fluorescence | | |
|---|---|---|---|
|  | Reading | Blank | Corrected |
| 0 | 18.8 | 16.3 | 2.5 |
| 5 | 26.0 | 22.0 | 4.0 |
| 10 | 34.0 | 28.4 | 5.6 |
| 20 | 40.1 | 32.2 | 7.9 |
| 30 | 45.7 | 35.5 | 10.2 |
| 40 | 48.0 | 38.2 | 9.8 |

The data demonstrate that an assay to determine the binding substance antigentamicin can be performed by the method of the present invention. The signal generated by the labeled monoclonal antigentamicin in the presence of the gentamicin-BSA macromolecular reagent is increased upon binding of goat antigentamicin to the reagent.

MS-1285

WHAT IS CLAIMED IS:

1. A homogeneous immunoassay method for determining an analyte in a test sample, comprising the steps of:

(a) combining said test sample with (i) a macromolecular reagent capable of binding with said analyte, and (ii) a labeled antireagent capable of competing with said analyte for binding with said macromolecular reagent, said labeled antireagent comprising a label which provides a detectable response which is measurably different when said labeled antireagent is bound with said macromolecular reagent compared to when not so bound, and

(b) measuring said detectable response as a function of said analyte in said sample.

2. The method of Claim 1 wherein said analyte is a binding substance for a molecule of molecular weight greater than about 10,000 daltons and wherein said macromolecular reagent comprises said molecule of molecular weight greater than about 10,000 daltons.

3. The method of Claim 2 wherein said binding substance is an antibody against a specific antigen.

4. The method of Claim 1 wherein said labeled antireagent is a labeled form of a monoclonal antibody or a fragment thereof.

MS-1285

5.    The method of Claim 1 wherein said test sample is also combined with a chemical detection system for the label comprised in said labeled antireagent, said label interacting with a member of said chemical detection system to provide a detectable response which is measurably different when said labeled antireagent is bound with said macromolecular reagent compared to when not so bound due to steric hindrance of access of said detection system member to said label.

6.    A reagent system for performing a homogeneous immunoassay to determine an analyte in a test sample, comprising:

(1) a macromolecular reagent capable of binding with said analyte, and

(2) a labeled antireagent capable of competing with said analyte for binding with said macromolecular reagent,

said labeled antireagent comprising a label which provides a detectable response which is measurably different when said labeled antireagent is bound with said macromolecular reagent compared to when not so bound.

7.    The reagent system of Claim 6 wherein said analyte is a binding substance for a molecule of molecular weight greater than about 10,000 daltons and wherein said macromolecular reagent comprises said molecule of molecular weight greater than about 10,000 daltons.

MS-1285

8. The reagent system of Claim 6 wherein said binding substance is an antibody against a specific antigen.

9. The reagent system of Claim 6 wherein said labeled antireagent is a labeled form of a monoclonal antibody or a fragment thereof.

10. The reagent system of Claim 6 wherein said test sample is also combined with a chemical detection system for the label comprised in said labeled antireagent, said label interacting with a member of said chemical detection system to provide a detectable response which is measurably different when said labeled antireagent is bound with said macromolecular reagent compared to when not so bound due to steric hindrance of access of said detection system member to said label.